# EUROPÄISCHE PATENTANMELDUNG

(11) **EP 0 827 957 A1**
(43) Veröffentlichungstag der Anmeldung: **11.03.1998**
(21) Anmeldenummer: 97115108.9
(22) Anmeldetag: 01.09.1997
(51) Int. Cl.: C07D 307/60

(54) **Verfahren zur Herstellung von Tetronsäurederivaten**

(30) Priorität: 04.09.1996 CH 2177/96
(71) Anmelder: LONZA AG, CH-3945 Gampel/Wallis (CH)
(72) Erfinder: Brieden, Walter, Dr., 3902 Glis (Kanton Wallis) (CH); Schröer, Josef, Dr., 3904 Naters (Kanton Wallis) (CH)
(74) Vertreter: KUHNEN, WACKER & PARTNER

(57) **Zusammenfassung**

Es wird ein neues Verfahren zur Herstellung von Tetronsäurederivaten der allgemeinen Formel 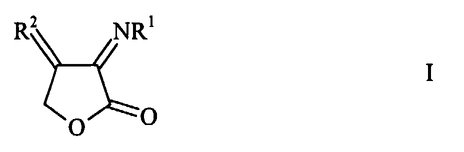 beschrieben, worin ein 4-Halogenacetessigester mit einer Base umgesetzt wird, die intermediär resultierende Tetronsäure entweder diazotiert oder nitrosyliert wird und die resultierende Tetronsäure gegebenenfalls mit einem Aminhydrohalogenid weiter umgesetzt wird.

Die resultierenden Tetronsäurederivate sind wichtige Zwischenprodukte zur Herstellung von (+)-Biotin, bzw. von Pharma- oder Agrowirkstoffen.

## Beschreibung

Die Erfindung betrifft ein neues Verfahren zur Herstellung von Tetronsäurederivaten der allgemeinen Formel 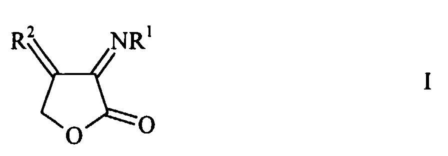 worin R¹ Hydroxy oder Phenylamino bedeutet und R² Oxo oder eine Gruppe NR¹ bedeutet, worin R¹ die genannte Bedeutung hat.
Tetronsäurederivate sind wichtige Zwischenprodukte für die Herstellung von u. a. (+)-Biotin oder von weiteren pharmazeutischen oder agrochemischen Wirkstoffen. Das ebenfalls als Vitamin H bekannte (+)-Biotin wird u.a. für pharmazeutische Anwendungen und auch als Zusatz in Futtermitteln verwendet.
Für die Herstellung von (+)-Biotin sind zahlreiche Synthesen veröffentlicht worden. Kommerzielle Bedeutung erlangt hat z.B. die in der EP-A 0 270 076 und in der EP-A 273 270 veröffentlichte Totalsynthese von (+)-Biotin ausgehend von Tetronsäure. Die Tetronsäure als Ausgangsprodukt dieser Totalsynthese konnte gemäss EP-A 0 153 615 ausgehend von einem 4-Chloracetessigsäureester durch Chlorierung mit Sulfurylchlorid zum 2,4-Dichloracetessigester, durch thermische Behandlung und Ringschluss zur 3-Chlortetronsäure und schliesslich durch Hydrierung der Chlortetronsäure zur Verfügung gestellt werden. Erst darauf konnte gemäss der genannten EP-A 0 270 076 in einem weiteren, nunmehr vierten Schritt die Diazotierung ansetzen.
Es ist offensichtlich, dass die genannte Bereitstellung der Ausgangsprodukte einerseits aufwendig, anderseits durch den Chlorierungsschritt mit Sulfurylchlorid auch ökologisch problembehaftet ist.
Die Aufgabe bestand folglich darin ein Verfahren zu entwickeln, dass diese Nachteile nicht beinhaltet.
Gelöst werden konnte diese Aufgabe mit dem neuen Verfahren gemäss Patentanspruch 1.

Gemäss dem erfindungsgemässen Verfahren wird in der ersten Stufe ein 4-Halogenacetessigsäureester der allgemeinen Formel 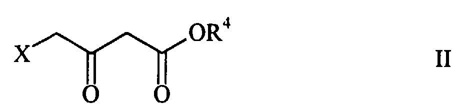 worin R⁴ eine C₁₋₆-Alkylgruppe und X ein Halogenatom bedeutet, zunächst mit einer Base in die Tetronsäure der Formel 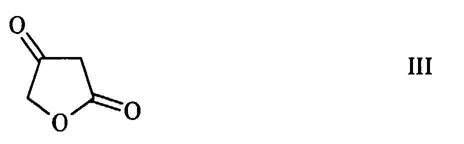 oder ein Salz davon, überführt.
C₁₋₆-Alkyl steht dabei zweckmässig für Methyl, Ethyl, n-Propyl, iso-Propyl, n-Butyl, sec.-Butyl, tert.-Butyl, Pentyl oder Hexyl und seine Isomeren. Bevorzugt wird eine C₁₋₄-Alkylgruppe verwendet. Unter Halogen wird Fluor, Chlor, Brom oder Jod, vorzugsweise Chlor verstanden. Bevorzugt eingesetzter 4-Halogenacetessigsäureester ist der 4-Chloracetessigsäureethylester.
Geeignete Basen sind Alkalihydroxide wie das Natrium- oder Kaliumhydroxid, Erdalkalihydroxide wie z. B. das Bariumhydroxid oder Stickstoffbasen.
Vorteilhaft erfolgt die Umsetzung in einem wässrigen System als Lösungsmittel, d.h. entweder in Wasser alleine oder in einer Mischung von Wasser mit einem geeigneten organischen Lösungsmittel wie z.B. Tetrahydrofuran, N,N-Dimethylformamid oder Acetonitril.
Die Umsetzung erfolgt zweckmässig bei einer Temperatur von -20 °C bis 100 °C, bevorzugt von 50 °C bis 100 °C. Bevorzugt wird dabei der 4-Halogenacetessigester der allgemeinen Formel II simultan mit der Base zudosiert bis das Reaktionsgemisch einen pH-Wert von 7 bis 13, bevorzugt von 7 bis 9 aufweist.

Die Tetronsäure der allgemeinen Formel III, bzw. ein Salz davon, wird zweckmässig ohne Isolation in die zweite Stufe überführt.

In der zweiten Stufe wird entweder mit einem Diazoniumsalz der allgemeinen Formel

R³N^{⊕}Y^{⊖} IVa

worin R³ Phenylimino bedeutet und Y ein Säureanion bedeutet, oder mit einer Nitrosylverbindung der allgemeinen Formel

NO^{⊕}Y^{⊖} IVb

worin Y die genannte Bedeutung hat, zu einem Tetronsäurederivat der allgemeinen Formel 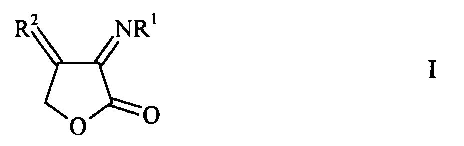 worin R¹ die genannte Bedeutung hat und R² Oxo bedeutet, umgesetzt.

Die Bereitstellung des Diazoniumsalzes gemäss der ersten Variante der Stufe 2 kann auf seit langem bekannte Art und Weise (vgl. EP-A 0 270 076) durch Umsetzung eines entsprechenden Anilins mit einer wässrigen Mineralsäure in Gegenwart eines Alkalinitrites erfolgen.
Die Phenyliminogruppe kann gegebenenfalls substituiert sein. Als allfällige Substituenten der Phenylgruppe können auftreten:
C₁₋₆-Alkyl d.h. zweckmässig Methyl, Ethyl, n-Propyl, iso-Propyl, n-Butyl, sec.-Butyl, tert.-Butyl, Pentyl oder Hexyl und seine Isomeren, C₁₋₆-Alkoxy, Aryl d.h. zweckmässig Phenyl oder Aryloxy wie zum Beispiel Phenoxy, Halogen oder Nitro.
Y als Säureanion hat zweckmässig die Bedeutung eines Anions einer Mineralsäure d.h. eines Halogenatoms wie z. B. Fluor, Chlor, Brom oder Jod, oder Hydrogensulfat oder BF₄. Bevorzugtes Diazoniumsalz ist das aus Anilin und Salzsäure hergestellte Benzoldiazoniumchlorid, worin R³ Phenylimino und Y Chlor bedeutet.

Die Bereitstellung der Nitrosylverbindung gemäss der zweiten Variante der Stufe 2 erfolgt auf bekannte Weise durch Umsetzung eines Alkalinitrites in Gegenwart einer Mineralsäure. Bevorzugte Nitrosylverbindung ist das aus Natriumnitrit und Salzsäure hergestellte Nitrosylchlorid (Formel IVb mit Y = Cl)

Die Umsetzung in der zweiten Stufe wird zweckmässig bei einer Temperatur zwischen -20 °C und 60 °C, vorzugsweise zwischen 10 °C und 30 °C durchgeführt.

Das nach der ersten Variante der Stufe 2 resultiernde Tetronsäurederivat der allgemeinen Formel I, mit der Bedeutung von R¹ = Phenylamino und R² = Oxo, kann isoliert werden und z. B. gemäss der Lehre der EP-A 0 270 076 zur Herstellung von (+)-Biotin eingesetzt werden, oder aber gemäss der dritten Stufe des erfindungsgemässen Verfahrens mit einem Aminhydrohalogenid der allgemeinen Formel

R¹NH₂·HX VI

worin R¹ und X die genannte Bedeutung haben, zum Endprodukt weiter umgesetzt werden.

Das nach der zweiten Variante der Stufe 2 resultierenden Tetronsäurederivat der allgemeinen Formel I mit der Bedeutung von R¹ = Hydroxy und R² = Oxo wird in der Regel ohne Isolation direkt in der dritten Stufe des erfindungsgemässen Verfahrens mit dem Aminhydrohalogenid der allgemeinen Formel VI umgesetzt.

Zweckmässig werden als Aminhydrohalogenide der allgemeinen Formel VI das Hydroxylaminhydrochlorid oder das Phenylhydrazinhydrochlorid verwendet. Die Umsetzung verläuft in der Regel im Lösungsmittelsystem der zweiten Stufe, oder aber, nach Lösungsmittelwechsel in einem geeigneten organischen Lösungsmittel wie z. B. einem aliphatischen Alkohol wie Methanol, Ethanol oder Propanol, vorzugsweise Ethanol. Die Reaktionstemperatur kann zwischen -20 °C und 100 °C variieren.
Die Isolation des Zielproduktes erfolgt auf übliche Art und Weise, z. B. durch Filtration.

### Beispiele:

### Beispiel 1:

### Herstellung von 3-Phenylazotetronsäure

Eine Lösung aus 2,2696 g 4-Chlor-acetessigsäure-ethylester (13,8 mmol) und 1,1030 g Natriumhydroxid (27,6 mmol) in 30 ml Wasser/THF = 2:1 wurde ca. 1 Stunde lang refluxiert, bis das Reaktionsgemisch ca. pH 8 erreicht hatte. Es wurde mit 40 ml Wasser verdünnt. Bei -10°C wurden zu 7 ml halbkonzentrierter wässriger Salzsäure (ca. 16% ig) zuerst tropfenweise 1,2844 g Anilin (13,8 mmol) und anschliessend eine Lösung von 0,9514 g Natriumnitrit (13,9 mmol) in 3 ml Wasser gegeben. Nach 10 min wurde diese blassgelbe, klare Lösung bei gleicher Temperatur mit einer gesättigten wässrigen Natriumacetatlösung titriert, bis ein Farbumschlag nach intensivgelb hin zu beobachten war. Gleichzeitig wurde die Lösung trübe.
Dieses Reagenz wurde nun zu der zuvor preparierten, auf -10°C gekühlten 4-Hydroxy-2(*5H*)-furanon-Natrium-Salz-Lösung (Natriumtetronat-Lösung) getropft. Ein dicker gelboranger Niederschlag fiel bei dieser Operation aus. Das Kühlbad wurde entfernt und man liess auf Raumtemperatur erwärmen. Anschliessend wurde 5 min auf 30°C erwärmt und dann filtriert. Der Niederschlag wurde zweimal mit je 30 ml Wasser und einmal mit 20 ml eiskaltem Methanol/Wasser = 1:1 gewaschen. Das Produkt wurde 18 Stunden bei 50°C im Vakuum getrocknet.
Es wurden 1,97 g 3-Phenylazotetronsäure erhalten.
- Ausbeute:: 70 %.
- ¹H-NMR (CDCl₃, 400 MHz) δ:: 4,70 (d, 2H);
7,36 (m, 1H);
7,48 (m, 2H):
7,58 (m, 2H):
13,12 (br, s, 0,5H);
14,00 (br, s, 0,5H).

### Beispiel 2:

### Herstellung von 3-Phenylazotetronsäure

In einem 10 l Doppelmantelrührwerk wurden 1996,0 g Wasser bei Rückfluss vorgelegt. Mittels Dosimat wurden innert 60 Minuten und simultan 164,60 g 4-Chloracetessigsäureethylester (1,00 Mol) und 226,60 g 30 % Natronlauge (2,00 Mol) zum Wasser zudosiert.
Nach der Zugabe wurde innerhalb von 90 Minuten von 96 °C auf 8 °C abgekühlt (pH-Wert 9,7). Dann wurde mit 0,5 g Essigsäure auf pH 7,2 gestellt, wobei sich die Lösung leicht grün verfärbte (Natriumtetronat-Lösung).
Zur Herstellung der Diazoniumsalz-Lösung wurden 192,60 g Wasser und 232,80 g 32 % Salzsäure bei 0 °C vorgelegt und dann mit 93,72 g Anilin (1,00 Mol) so versetzt, dass die Temperatur 0 °C nicht überstieg. Es entstand dabei eine weisse Suspension. Zu dieser Suspension wurde eine Lösung von 69,40 g Natriumnitrit (1,00 Mol) in 100,3 g Wasser so zugegeben, dass die Temperatur zwischen -10 °C und 0 °C blieb. Die entstandene gelbe Lösung wurde 10 Minuten bei 0 °C gerührt und durch Zugabe von 281 g 10 % Natriumacetat-Lösung gepuffert.
Diese Diazoniumsalz-Lösung wurde nun innert 5 Minuten zur 5 °C kalten Natriumtetronat-Lösung zudosiert. Es fiel sofort ein gelber Feststoff aus, der nach 30 Minuten Rühren bei 5 °C abfiltriert und mit 727 g Wasser und 500 g kalten Methanol gewaschen wurde. Das gelbe Produkt wurde bei 50 °C im Vakuum getrocknet.
- Ausbeute:: 178,8 g Titelprodukt aus 1. Filtration (87,5 % Ausbeute)
7,5 g Titelprodukt aus Mutterlauge (3,6 % Ausbeute)

### Beispiel 3:

### Herstellung einer 0,5 M Natriumtetronat-Lösung

In einem 2 l Doppelmantelrührwerk wurden 1620 g Wasser bei Rückfluss vorgelegt. Mittels Dosimat wurde innert 60 Minuten und simultan 164,40 g 4-Chloracetessigsäureethylester (1,00 Mol) und 266,60 g 30 % Natronlauge (2,00 Mol) zum siedenden Wasser zudosiert. Nach der Zugabe wurde direkt von 96 °C auf 22 °C abgekühlt und mit Wasser genau auf ein Volumen von 2 l verdünnt (0,5 M Natriumtetronat-Lösung).
- ¹³C-NMR (H₂O, DMSO insert) δ:: 194,47 (C2);
183,14 (C4);
80,34 (C3);
70,95 (C5);
56,58 (EtOH);
15,99 (EtOH).

### Beispiel 4:

### Herstellung von 3-Hydroxyimino-2-oxo-4-phenylhydrazonotetrahydrofuran

30 ml Natriumtetronat-Lösung (30 mmol) wurden bei Raumtemperatur vorgelegt und darin 2,07 g Natriumnitrit (30 mmol) gelöst. Zu der klaren Lösung wurden innert 30 Minuten 6 ml 5M Salzsäure (30 mmol) zudosiert. Die Temperatur wurde zwischen 20 °C - 25 °C gehalten. Nach der Salzsäure-Zugabe liess man noch 15 Minuten bei Raumtemperatur weiterrühren. Diese Lösung wurde danach innert 35 Minuten zu einer 0 °C kalten Suspension von 5,20 g Phenylhydrazin-hydrochlorid (36 mmol) in 60 ml Wasser zugegeben. Dabei fiel ein oranger Feststoff aus. Nach der Zugabe liess man noch 2 Stunden bei 0 °C weiterrühren, filtrierte den Feststoff ab, wusch mit wenig Wasser und trocknete im Vakuum bei 40 °C.
- Ausbeute:: 4,70 g Titelprodukt (64 % Ausbeute)
- Schmelzpunkt:: 191,0 °C - 191,5 °C
- ¹H-NMR DMSO-d₆) δ:: 5,00 (s, 2H, CH₂);
6,90 (t, 1H, CHₐᵣ);
7,22 (d, 2H, CHₐᵣ);
7,29 (t, 2H, CHₐᵣ);
10,03 (s, 1H, NH);
13,28 (s, 1H, NOH).

### Beispiel 5:

### Herstellung von 3,4-Bis[hydroxyimino]-2-oxotetrahydrofuran

50 ml Natriumtetronat-Lösung (25 mmol) wurden bei Raumtemperatur vorgelegt und darin 3,45 g Natriumnitrit gelöst. Zu der klaren Lösung wurden innert 30 Minuten 10 ml 5M Salzsäure (50 mmol) zudosiert. Die Temperatur wurde zwischen 20 °C - 25 °C gehalten. Nach der Salzsäure-Zugabe liess man noch 15 Minuten bei Raumtemperatur weiterrühren. Diese Lösung wurde danach bei Raumtemperatur innert 35 Minuten zu einer Lösung von 6,85 g Hydroxylamin-hydrochlorid (100 mmol) in 70 ml Wasser zugegeben. Nach 2,5 Stunden Weiterrühren bei Raumtemperatur fiel ein beiger Feststoff aus. Danach wurde auf 0 °C abgekühlt, der Feststoff abfiltriert, mit wenig Wasser gewaschen und im Vakuum bei 40 °C getrocknet.
- Ausbeute:: 1,40 g Titelprodukt (38 % Ausbeute)
- Schmelzpunkt:: 169,8 °C - 170,2 °C
- ¹H-NMR (DMSO-d₆) δ:: 4,98 (s, 2H, CH₂);
12,40 (s, 1H, NOH);
13,90 (s, 1H, NOH).

### Beispiel 6:

### Herstellung von 2-Oxo-3,4-bis[phenylhydrazono]-tetrahydrofuran

Es wurden 4,08 g (20 mmol) Phenylazotetronsäure, 11,56 g (80 mmol) Phenylhydroxylaminhydrochlorid 5,60 g (69 mmol) Natriumacetat und 200 ml Ethanol vorgelegt und für 7 h zum Rückfluss erhitzt. Die entstandene dicke, rötliche Suspension wurde auf Raumtemperatur abgekühlt und anschliessend abfiltriert. Der rötliche voluminöse Feststoff wurde 5mal mit 50 ml Wasser gewaschen und zum Schluss noch 2mal mit 50 ml Ethanol nachgewaschen. Der rote Feststoff wurde im Vakuumtrockenschrank bei 40 °C getrocknet.
- Ausbeute:: 5,21 g rötlicher Feststoff (88% Ausbeute)
- ¹H-NMR (400 MHz, DMSO-d₆) δ:: 12,40 (s, 1H, NH);
10,10 (s, 1H, NH);
7,44 (m, 2H, CH_{arom.});
7,38 (m, 4H, CH_{arom.});
7,20 (d, 2H, CH_{arom.});
7,12 (t, 1H, CH_{arom.});
6,94 (t, 1H, CH_{arom.});
5,18 (s, 2H, CH_{arom.}).
- ¹³C-NMR (100 MHz, DMSO-d₆) δ:: 166,05 (C2);
143,76 (C4);
142,13;
139,34 (2 Cᵢₚₛₒ);
129,70;
129,41;
123,57;
120,81;
114,51;
112,80 (10 CH_{arom.});
122,43 (C3);
65,04 (C5).

### Beispiel 7:

### Herstellung von 4-Hydroxyimino-2-oxo-3-phenylhydrazonotetrahydrofuran

Es wurden 4,08 g (20 mmol) Phenylazotetronsäure, 5,60 g (80 mmol) Hydroxylaminhydrochlorid, 5,60 g (69 mmol) Natriumacetat und 200 ml Ethanol vorgelegt und für 3 h zum Rückfluss erhitzt. Die entstandene gelbe Suspension wurde anschliessend auf Raumtemperatur abgekühlt und das Lösungsmittel am Rotavapor abdestilliert. Zu diesem Rückstand gab man 100 ml Wasser und filtrierte den Feststoff ab. Es wurde noch 3mal mit 50 ml Wasser nachgewaschen und der gelbe Feststoff wurde danach aus 200 ml Ethanol heiss umkristallisiert. Die Mutterlauge wurde abgekühlt und der ausgefallene Feststoff abfiltriert, mit 50 ml Ethanol gewaschen und im Vakuumtrockenschrank bei 40 °C getrocknet.
- Ausbeute:: 2,41 g gelber Feststoff (55% Ausbeute)
- ¹H-NMR (400 MHz, DMSO-d₆) δ:: 12,32 (s, 1H, OH oder NH);
11,64 (s, 1H, OH oder NH);
7,44 (t, 2H, CH_{arom.});
7,34 (d, 2H, CH_{arom.});
7,16 (t, 1H, CH_{arom.});
5,06 (s, 2H, CH₂O)
- ¹³C-NMR (100 MHz, DMSO-d₆) δ:: 165,49 (C2);
151,74 (C4);
141,70 (Cᵢₚₛₒ);
129,65;
124,31;
114,80 (5 CH_{arom.});
120,87 (C3);
64,31 (C5).

## Patentansprüche

1. Verfahren zur Herstellung von Tetronsäurederivaten der allgemeinen Formel 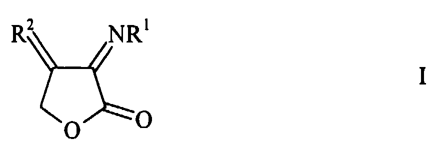 worin R¹ Hydroxy oder gegebenenfalls substituiertes Phenylamino bedeutet und R² Oxo oder eine Gruppe NR¹ bedeutet, worin R¹ die genannte Bedeutung hat, dadurch gekennzeichnet, dass ein 4-Halogenacetessigester der allgemeinen Formel 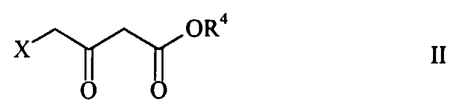 worin R⁴ eine C₁₋₆-Alkylgruppe und X ein Halogenatom bedeutet, in der ersten Stufe in Gegenwart einer Base in die Tetronsäure der Formel 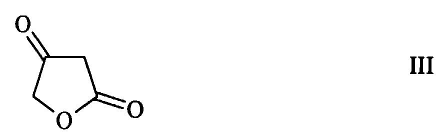 bzw. ein Salz davon überführt wird, dieses in der zweiten Stufe mit einem Diazoniumsalz der allgemeinen Formel
R³N^{⊕}Y^{⊖} IVa
worin R³ gegebenenfalls substituiertes Phenylimino bedeutet und Y ein Säureanion bedeutet, oder mit einer Nitrosylverbindung der allgemeinen Formel
NO^{⊕}Y^{⊖} IVb
worin Y die genannte Bedeutung hat zu einem Tetronsäurederivat der allgemeinen Formel 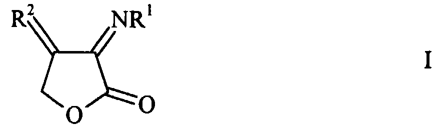 worin R¹ die genannte Bedeutung hat und R² Oxo bedeutet, umgesetzt wird, letzteres isoliert wird oder in einer dritten Stufe weiter umgesetzt wird mit einem Aminhydrohalogenid der allgemeinen Formel
R¹NH₂·HX VI
worin R¹ und X die genannte Bedeutung haben.

2. Verfahren nach Patentanspruch 1, dadurch gekennzeichnet, dass als Base ein Alkalihydroxid verwendet wird.

3. Verfahren nach Patentanspruch 1 oder 2, dadurch gekennzeichnet, dass die Umsetzung in der ersten Stufe bei einer Temperatur von -20 °C bis 100 °C so durchgeführt wird, dass der 4-Halogenacetessigester der allgemeinen Formel II simultan mit der Base zudosiert wird, bis das Reaktionsgemisch einen pH-Wert von 7 bis 13 aufweist.

4. Verfahren nach einem der Patentansprüche 1 bis 3, dadurch gekennzeichnet, dass die Tetronsäure der Formel III nicht isoliert wird.

5. Verfahren nach einem der Patentansprüche 1 bis 4, dadurch gekennzeichnet, dass die Umsetzung in der zweiten Stufe bei einer Temperatur von -20 °C bis 60 °C erfolgt.

6. Verfahren nach einem der Patentansprüche 1 bis 5, dadurch gekennzeichnet, dass die Umsetzung in der dritten Stufe bei einer Temperatur von -20 °C bis 100 °C erfolgt.

7. Verfahren nach Patentanspruch 1 zur Herstellung von einem Tetronsäurederivat der allgemeinen Formel I, worin R¹ gegebenenfalls substituiertes Phenylamino und R² Oxo bedeutet, dadurch gekennzeichnet, dass ein 4-Halogenacetessigester der allgemeinen Formel 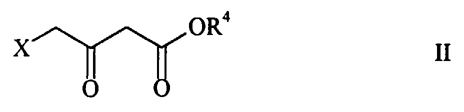 worin R⁴ eine C₁₋₆-Alkylgruppe und X ein Halogenatom bedeutet, in der ersten Stufe in Gegenwart einer Base in die Tetronsäure der Formel 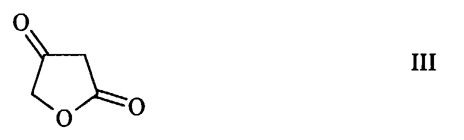 bzw. ein Salz davon überführt wird und in der zweiten Stufe mit einem Diazoniumsalz der allgemeinen Formel
R³N^{⊕}Y^{⊖} IVa
worin R³ gegebenenfalls substituiertes Phenylimino bedeutet und Y ein Säureanion bedeutet, in das Zielprodukt überführt wird.
